# EUROPÄISCHE PATENTSCHRIFT

(11) **EP 0 353 668 B1**
(45) Veröffentlichungstag und Bekanntmachung des Hinweises auf die Patenterteilung: **19.10.1994**
(21) Anmeldenummer: 89114031.1
(22) Anmeldetag: 29.07.1989
(51) Int. Cl.: C07D 213/81, A61K 31/44

(54) **Verbessertes Verfahren zur Herstellung von N,N-Bis(alkoxyalkyl)-pyridin -2,4-dicarbonsäurediamiden**
Process for the preparation of N,N-bis(alkoxyalkyl) diamides of pyridine-2,4-dicarboxylic acid
Procédé pour la préparation de N,N-bis(alkoxyalkyle) diamides de l'acide pyridine 2,4-dicarboxylique

(30) Priorität: 04.08.1988 DE 3826471; 19.08.1988 DE 3828140
(43) Veröffentlichungstag der Anmeldung: 07.02.1990
(73) Patentinhaber: HOECHST AKTIENGESELLSCHAFT, 65926 Frankfurt am Main (DE)
(72) Erfinder: Baader, Ekkehard, Dr., D-6240 Königstein Taunus (DE); Burghard, Harald, Dr., D-6384 Schmitten 3 (DE); Bickel, Martin, Dr., D-6380 Bad Homburg (DE); Günzler-Pukall, Volkmar, Dr., D-3550 Marburg (DE)

(56) Entgegenhaltungen:
- EP-A- 0 278 453
- GB-A- 1 235 348

## Beschreibung

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren, wie α,α'-Dipyridyl zu einer Hemmung der C1_{q}-Biosythese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238, 625-633, 1987).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Es wurde bereits vorgeschlagen (in der älteren deutschen Patentanmeldungen P 37 03 959.8, P 37 03 962.8, P 37 03 963.6 und P 37 07 429.6) gemischte Ester/Amide, höher alkylierte Diester und Diamide der Pyridin-2,4- und -2,5-dicarbonsäure einzusetzten, um die Kollagenbisoynthese im Tiermodell wirksam zu hemmen.

In der älteren deutschen Patentanmeldung P 37 03 959.8 wird unter anderem die Synthese von
N,N'-Bis(2-methoxyethyl)-pyridin-2,4-dicarbonsäurediamid (III) 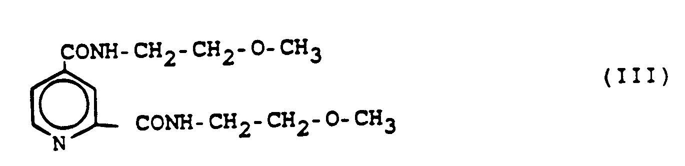
und
N,N'-Bis(3-isopropoxypropyl)-pyridin-2,4-dicarbonsäurediamid (IV) 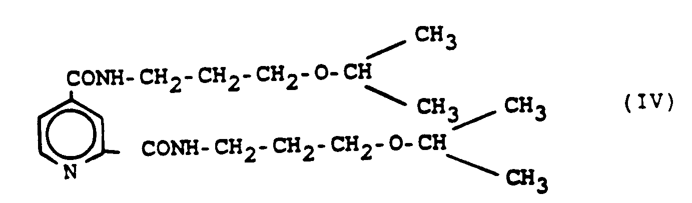
vorgeschlagen. Diese Verbindungen fallen jedoch entweder nur in sehr geringen Ausbeuten an (III ca. 5% und IV ca. 59% Ausbeute bezogen auf eingesetzte Pyridin-2,4-dicarbonsäure) oder die vorgeschlagenen Verfahren zu ihrer Herstellung sind verfahrenstechnisch für eine Massenproduktion wenig vorteilhaft, da die Herstellung der Verbindungen zum einen zu viele Syntheseschritte umfaßt und zum anderen die Produktion recht energieaufwendig ist (Abdestillieren von SOCl₂, Abrauchen mit Toluol, Zutropfen bei -30 bis -20°C) und schließlich auch noch ein chromatographischer Reinigungsschritt nötig ist.

Es wurde nun ein Verfahren gefunden, daß die Synthese von Verbindungen der Formel I 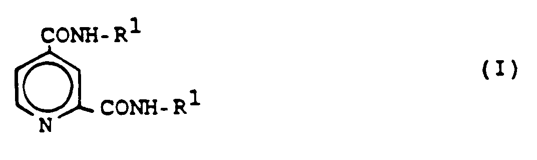
worin
- R¹: C₁-C₄-Alkyl bedeutet, welches mit C₁-C₄-Alkoxy monosubstituiert ist,
in Ausbeuten von über 70 % (bezogen auf eingesetzte Pyridin-2,4-dicarbonsäure) ermöglicht und welches im Vergleich zu dem in der deutschen Patentanmeldung P 37 03 959.8 vorgeschlagenen Verfahren wesentlich weniger energieaufwendig ist und vor allem ohne chromatographische Reinigung auskommt, da das Endprodukt direkt als Feststoff anfällt.
Die ausgewählten Verbindungen zeigen eine sehr gute enterale Resorbierbarkeit, auf Grund derer sie sich besonders gut für eine orale Applikationsform eignen. Die pharmakologische Wirksamkeit der Verbindungen der Formel I entspricht den in der deutschen Patentanmeldung P 37 03 959.8 vorgeschlagenen Verbindungen.

Die Erfindung betrifft somit:
Ein Verfahren zur Herstellung von
N,N'-Bis-(alkoxyalkyl)-pyridin-2,4-dicarbonsäurediamiden der Formel I 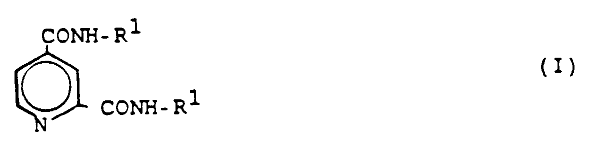
worin
- R¹: C₁-C₄-Alkyl bedeutet, welches mit C₁-C₄-Alkoxy monosubstituiert ist,
wobei man Pyridin-2,4-dicarbonsäurechlorid mit einem Alkoxyalkylamin umsetzt, das dadurch gekennzeichnet ist, daß man zunächst
A) Pyridin-2,4-dicarbonsäure in Toluol suspendiert, bei Raumtemperatur mit einem 2-3-fach molaren Überschuß SOCl₂ versetzt, bis zur Beendigung der Gasentwicklung auf 90-110°C erhitzt, die klare Lösung eindampft und das erhaltene Zwischenprodukt in Dioxan löst und daß man
B) die 4-fach molare Menge an Alkoxyalkylamin der Formel II

   H₂N-(R²)-OR³ (II)

   worin
   - R²: C₁-C₄-Alkylen und
   - R³: C₁-C₄-Alkyl bedeutet
   mit Dioxan versetzt
und daß man dann bei einer Temperatur von -5 bis +5°C entweder die gemäß A) hergestellt Lösung zu der gemäß B) hergestellten Lösung tropft oder die gemäß B) hergestellte Lösung zu der gemäß A) hergestellten Lösung tropft, anschließend bei Raumtemperatur nachreagieren läßt und dann das Reaktionsprodukt ansäuert und daß man anschließend die organische Phase abtrennt, mit Wasser wäscht, trocknet und dann vom Lösungsmittel befreit.

Die genannten Alkyl-, und Alkoxy-Reste mit 3 und mehr Kohlenstoffatomen können sowohl geradkettig als auch verzweigt sein. Unter einem C₁-C₄-Alkylen-Rest werden Methylen, Ethylen, n-Propylen oder n-Butylen verstanden, als auch alkylsubstituierte Alkylene. Beispiele hierfür sind Methylmethylen, Ethylmethylen, n-Propylmethylen, i-Propylmethylen, Methylethylen oder Ethylethylen.

Bevorzugt sind Verfahren, in denen mindestens eine der folgenden Bedingungen erfüllt ist: R¹ bedeutet C₁-C₃-Alkyl, welches mit C₁-C₃-Alkoxy monosubstituiert ist, insbesondere 2-Methoxyethyl; R² bedeutet C₁-C₃-Alkylen, insbesondere Ethylen; R³ bedeutet C₁-C₃-Alkyl, insbesondere Methyl.

Bei dem erfindungsgemäßen Verfahren wird die als Ausgangssubstanz käuflich erhältliche Pyridin-2,4-dicarbonsäure in Toluol suspendiert und bei Raumtemperatur mit SOCl₂ versetzt. Bezogen auf die eingesetzte molare Menge an Pyridin-2,4-dicarbonsäure werden 2-3-fach molare Mengen an SOCl₂ eingesetzt, bevorzugt 2-fach molare Mengen. Die erhaltene Reaktionsmischung wird auf 90-110°C, vorzugsweise auf 100°C, solange erhitzt, bis keine HCl-Gasentwicklung mehr zu beobachten ist und eine klare Lösung entstanden ist. Anschließend wird die Lösung -vorzugsweise im Hochvakuum (bis ca. 10⁻³ Torr)-eingedampft, und das erhaltene Carbonsäurechlorid dann in Dioxan gelöst.

Bezogen auf die eingesetzte molare Menge an Pyridin-2,4-dicarbonsäure wird nun die 4-fach molare Menge an käuflichem Alkoxyalkylamin (Verbindung der Formel II) in Dioxan gelöst. Bevorzugt wird dann das Alkoxyalkylamin zu dem gelösten Pyridin-2,4-dicarbonsäurechlorid getropft. Es ist jedoch auch möglich, die Lösung des Carbonäurechlorids zu der Lösung des Alkoxyalkylamins zu tropfen. In beiden Fällen erfolgt die Zugabe bei einer Temperatur von -5 bis +5°C, bevorzugt bei 0°C. Die Reaktionsmischung, läßt man dann auf Raumtemperatur erwärmen und rührt noch 2-5 Stunden bevorzugt 3 Stunden nach. Das erhaltene Produkt wird anschließend angesäuert, um überschüssiges Alkoxyalkylamin vom gewünschten Produkt abzutrennen. Das Ansäuern kann beispielsweise mit 0,2 molarer Citronensäure erfolgen. Die organische Phase wird dann abgetrennt und mit Wasser gewaschen. Anschließend wird die organischen Phase getrocknet -vorzugsweise über Magnesiumsulfat- und schließlich vom Lösungsmittel befreit. Beim Entfernen des Lösungsmittels fällt das Produkt -N,N'-Bis(alkoxyalkyl)-pyridin-2,4-dicarbonsäurediamid- als weißer Festkörper aus.

Es wurde festgestellt, daß die Verbindungen der Formel I außergewöhnlich gute enterale Resorbierbarkeiten aufweisen. Die Resorbierbarkeit wurde an Wistarratten untersucht, denen N,N'-Bis-(2-methoxy ethyl)pyridin-2,4-dicarbonsäureamid intragastral verabreicht wurde. Der Serumspiegel sank in den ersten Stunden nach Verabreichung der Substanz ab und ereichte nach ca. 5 Stunden ein nur noch leicht abfallendes Platteau. Aus dem zunächst sehr hohen Serumspiegel direkt nach Verabreichung der Substanz läßt sich auf die gute Resorbierbarkeit der Substanz schließen.

Im folgenden ist die Erfindung anhand von Beispielen näher erläutert.

### Enterale Resorbierbarkeit

Weibliche Wistarratten von ca. 150 g Körpergewicht erhalten 50 mg/kg N,N'-Bis(2-methoxyethyl)pyridin-2,4-dicarbonsäurediamid mittels Schlundsonde intragastral verabreicht. Es werden nach 0; 0,25; 0,5; 1; 3; 4; 5; 6; 8 und 14 Stunden jeweils 4 Ratten narkotisiert und über die Vena Cava entblutet. Das Blut wird sofort zentrifugiert und N,N'-Bis(2-methoxyethyl)pyridin-2,4-dicarbonsäurediamid mit Ether aus dem Serum extrahiert. Nach Verdampfen des Ethers wird der Rückstand in 100 ml Fliessmittel aufgenommen. Das Fliessmittel besteht aus 0,05 M Phosphorsäure und Acetonitril (4:1).
Von dieser Probe werden 50 µl in eine HPLC-Säule injiziert. Die Detektion erfolgt bei UV 200 nm und einer Retentionszeit von 2,2 min. Die Ergebnisse sind in der Tabelle 1 und der Figur 1 dokumentiert.

**Tabelle 1**

| Serumspiegel von N,N'-Bis(2-methoxyethyl)pyridin-2,4-dicarbonsäurediamid nach Gabe von 50 mg/kg p.o. | | | |
|---|---|---|---|
| Zeit (h) | Serumspiegel µg/ml | | |
| | x̅ | SD | SEM |
| 0.25 | 38.7 | ± 2.2 | 1.6 |
| 0.5 | 32.4 | ± 3.1 | 2.1 |
| 1 | 15.6 | ± 1.5 | 1 |
| 3 | 0.7 | ± 0.07 | 0.04 |
| 4 | 3.3 | ± 2.2 | 1.6 |
| 5 | 0.5 | ± 0.3 | 0.2 |
| 6 | 0.6 | ± 0.1 | 0.1 |
| 8 | 0.5 | ± 0.3 | 0.2 |
| 14 | 0.3 | ± 0.2 | 0.1 |
| x̅ = Mittelwert aus 4 Messungen SD = Standardabweichung SEM = Standard error of the mean | | | |

### Beispiel 1

### Herstellung von N,N'-Bis(2-methoxyethyl)pyridin-2,4-dicarbonsäurediamid

10 g Pyridin-2,4-dicarbonsäure werden in 150 ml Toluol suspendiert und mit 9,1 ml SOCl₂ bei Raumtemperatur versetzt. Das Reaktionsgemisch wird dann so lange auf 100 °C erhitzt, bis die Lösung klar ist und keine Gasentwicklung mehr zu beobachten ist. Die Lösung wird im Hochvakuum eingedampft, in 100 ml Dioxan gelöst und bei 0°C 21 ml 2-Methoxyethylamin in 100 ml Dioxan zugetropft. Nach 3 Stunden Rühren bei Raumtemperatur wird die Lösung mit 0,2 M Citronensäure versetzt (pH 4), die organische Phase abgetrennt und mit Wasser gewaschen. Nach Trocknen mit Magnesiumsulfat wird die Lösung vom Lösungsmittel befreit und ergibt 12,8 g (76 % Ausbeute bezogen auf Pyridin-2,4-dicarbonsäure) eines weißen Festkörpers.
Fp. 86 °C

## Patentansprüche

1. Verfahren zur Herstellung von N,N'-Bis-(alkoxyalkyl)pyridin-2,4-dicarbonsäurediamiden der Formel I 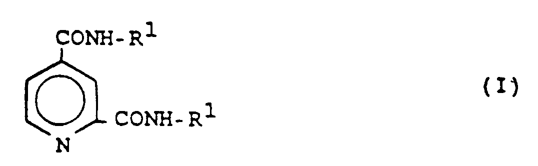 worin
R¹ C₁-C₄-Alkyl bedeutet, welches mit C₁-C₄-Alkoxy monosubstituiert ist
wobei man Pyridin-2,4-dicarbonsäurechlorid mit einem Alkoxyalkylamin umsetzt, dadurch gekennzeichnet, daß man zunächst
A) Pyridin-2,4-dicarbonsäure in Toluol suspendiert, bei Raumtemperatur mit einem 2-3-fach molaren Überschuß SOCl₂ versetzt, bis zur Beendigung der Gasentwicklung auf 90-110°C erhitzt, die klare Lösung eindampft und das erhaltene Zwischenprodukt in Dioxan löst und daß man
B) die 4-fach molare Menge an Alkoxyalkylamin der Formel II
H₂N-(R²)-OR³ (II)
worin
R² C₁-C₄-Alkylen und
R³ C₁-C₄-Alkyl bedeutet
mit Dioxan versetzt
und daß man dann bei einer Temperatur von -5 bis +5°C entweder die gemäß A) hergestellte Lösung des Zwischenproduktes zu der gemäß B) hergestellten Lösung tropft oder die gemäß B) hergestellte Lösung zu der gemäß A) hergestellten Lösung des Zwischenproduktes tropft, anschließend bei Raumtemperatur nachreagieren läßt und dann das Reaktionsprodukt ansäuert und daß man anschließend die organische Phase abtrennt, mit Wasser wäscht, trocknet und dann vom Lösungsmittel befreit.

2. Verfahren nach Anspruch 1, dadurch gekennzeichnet, daß man einen 2-fach molaren überschuß an SOCl₂ verwendet.

3. Verfahren nach Anspruch 1 und/oder 2, dadurch gekennzeichnet, daß die Pyridin-2,4-dicarbonsäure und das SOCl₂ auf 100°C erhitzt werden.

4. Verfahren nach einem oder mehreren der Ansprüche 1 bis 3, dadurch gekennzeichnet, daß die Lösung, hergestellt gemäß A) zu der Lösung, hergestellt gemäß B) oder die Lösung hergestellt gemäß B) zu der Lösung, hergestellt gemäß A) bei einer Temperatur von 0°C getropft wird.

5. Verfahren nach einem oder mehreren der Ansprüche 1 bis 4, dadurch gekennzeichnet, daß man die beiden Lösungen, hergestellt gemäß A) und hergestellt gemäß B) 3 Stunden bei Raumtemperatur nachreagieren läßt.

6. Verfahren nach einem oder mehreren der Ansprüche 1 bis 5, dadurch gekennzeichnet, daß mindestens eine der nachfolgenden Bedingungen erfüllt ist:
R¹ bedeutet C₁-C₃-Alkyl, welches mit C₁-C₃-Alkoxy monosubstituiert ist
R² bedeutet C₁-C₃-Alkylen und
R³ bedeutet C₁-C₃-Alkyl.

7. Verfahren nach einem oder mehreren der Ansprüche 1 bis 6, dadurch gekennzeichnet, daß mindestens eine der nachfolgenden Bedingungen erfüllt ist:
R¹ bedeutet 2-Methoxyethyl
R² bedeutet Ethylen und
R³ bedeutet Methyl.

## Claims

1. A process for the preparation of N,N'-bis-(alkoxyalkyl)pyridine-2,4-dicarboxamides of the formula I 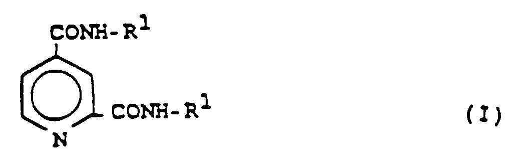 in which
R¹ denotes C₁-C₄-alkyl which is monosubstituted by C₁-C₄-alkoxy,
entailing reaction of pyridine-2,4-dicarbonyl chloride with an alkoxyalkylamine, which comprises firstly
A) pyridine-2,4-dicarboxylic acid being suspended in toluene, a 2-3-fold molar excess of SOCl₂ being added at room temperature, the mixture being heated at 90-110°C until evolution of gas ceases, the clear solution being evaporated, and the resulting intermediate being dissolved in dioxane, and
B) dioxane being added to 4 times the molar amount of alkoxyalkylamine of the formula II
H₂N-(R²)-OR³ (II)
in which
R² denotes C₁-C₄-alkylene and
R³ denotes C₁-C₄-alkyl, and
then, at a temperature of -5 to +5°C, either the solution of the intermediate prepared as in A) being added dropwise to the solution prepared as in B), or the solution prepared as in B) being added dropwise to the solution of the intermediate prepared as in A), reaction subsequently being allowed to take place at room temperature, and then the reaction product being acidified, and subsequently the organic phase being separated off, washed with water, dried and then freed of solvent.

2. The process as claimed in claim 1, wherein a 2-fold molar excess of SOCl₂ is used.

3. The process as claimed in claim 1 and/or 2, wherein the pyridine-2,4-dicarboxylic acid and the SOCl₂ are heated at 100°C.

4. The process as claimed in one or more of claims 1 to 3, wherein the solution prepared as in A) is added dropwise to the solution prepared as in B), or the solution prepared as in B) is added dropwise to the solution prepared as in A), at a temperature of 0°C.

5. The process as claimed in one or more of claims 1 to 4, wherein the two solutions prepared as in A) and prepared as in B) are subsequently allowed to react at room temperature for 3 hours.

6. The process as claimed in one or more of claims 1 to 5, wherein at least one of the following conditions is met:
R¹ denotes C₁-C₃-alkyl which is monosubstituted by C₁-C₃-alkoxy,
R² denotes C₁-C₃-alkylene and
R³ denotes C₁-C₃-alkyl.

7. The process as claimed in one or more of claims 1 to 6, wherein at least one of the following conditions is met:
R¹ denotes 2-methoxyethyl,
R² denotes ethylene and
R³ denotes methyl.

## Revendications

1. Procédé pour la préparation de N,N'-bis(alcoxyalkyl)pyridine-2,4-dicarboxamides de formule I 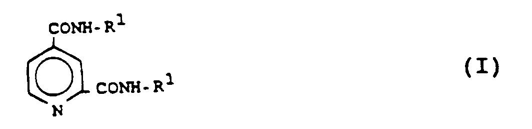 dans laquelle
R¹ représente un radical alkyle en C₁-C₄ qui est monosubstitué par un groupe alcoxy en C₁-C₄,
dans lequel on fait réagir du dichlorure d'acide pyridine-2,4-dicarboxylique avec une alcoxyalkylamine, qui est caractérisé en ce que
A) on met en suspension de l'acide pyridine-2,4-dicarboxylique dans du toluène, on y ajoute, à la température ambiante, un excès 2-3 fois molaire de SOCl₂, on chauffe le mélange à 90-110°C jusqu'à la fin du dégagement de gaz, on évapore la solution limpide et on dissout dans du dioxanne le produit intermédiaire obtenu, et en ce que
B) on ajoute du dioxanne à la quantité 4 fois molaire d'une alcoxyalkylamine de formule II
H₂N-(R²)-OR³ (II)
dans laquelle
R² représente un groupe alkylène en C₁-C₄ et
R³ représente un groupe alkyle en C₁-C₄,
et en ce que ensuite, à une température de -5 à +5°C, soit on ajoute goutte à goutte la solution préparée selon A) à la solution préparée selon B), soit on ajoute goutte à goutte la solution selon B) à la solution préparée selon A), puis on fait réagir le mélange à la température ambiante et on acidifie ensuite le produit de réaction, et en ce que la phase organique est ensuite isolée , lavée avec de l'eau, séchée en ensuite séparée d'avec le solvant.

2. Procédé selon la revendication 1, caractérisé en ce que l'on utilise un excès 2 fois molaire de SOCl₂.

3. Procédé selon la revendication 1 et/ou la revendication 2, caractérisé en ce que l'on chauffe à 100°C l'acide pyridine-2,4-dicarboxylique et le SOCl₂.

4. Procédé selon une ou plusieurs des revendications 1 à 3, caractérisé en ce que l'on ajoute, à une température de 0°C, la solution préparée selon A) à la solution selon B), ou la solution préparée selon B) à la solution préparée selon A).

5. Procédé selon une ou plusieurs des revendications 1 à 4, caractérisé en ce que l'on fait réagir pendant 3 heures à la température ambiante les deux solutions, celle préparée selon A) et celle préparée selon B).

6. Procédé selon une ou plusieurs des revendications 1 à 5, caractérisé en ce qu'au moins l'une des conditions suivantes est remplie:
R¹ représente un radical alkyle en C₁-C₃ qui est monosubstitué par un groupe alcoxy en C₁-C₃,
R² représente un radical alkylène en C₁-C₃ et
R³ représente un radical alkyle en C₁-C₃.

7. Procédé selon une ou plusieurs des revendications 1 à 6, caractérisé en ce qu'au moins l'une des conditions suivantes est remplie:
R¹ représente le groupe 2-méthoxyéthyle,
R² représente le groupe éthylène et
R³ représente le groupe méthyle.
